# EUROPÄISCHE PATENTSCHRIFT

(11) **EP 3 381 423 B1**
(45) Veröffentlichungstag und Bekanntmachung des Hinweises auf die Patenterteilung: **25.03.2020**
(21) Anmeldenummer: 18163434.6
(22) Anmeldetag: 22.03.2018
(51) Int. Cl.: A61F 13/10, A61F 5/01

(54) **MANSCHETTE ZUR ORTHETISCHEN BEHANDLUNG**
CUFF FOR ORTHOTIC TREATMENT
MANCHETTE DESTINÉE AU TRAITEMENT ORTHÉTIQUE

(30) Priorität: 30.03.2017 DE 202017101866 U
(43) Veröffentlichungstag der Anmeldung: 03.10.2018
(73) Patentinhaber: Röder, Ulrike, 21337 Lüneburg (DE)
(72) Erfinder: Röder, Harry, 21388 Soderstorf (DE)
(74) Vertreter: Meyer, Ludgerus

(56) Entgegenhaltungen:
- US-A- 4 489 716
- US-A1- 2015 101 095
- US-A1- 2017 042 720
- US-B1- 7 074 202

## Beschreibung

Die Erfindung betrifft eine Manschette zur orthetischen Behandlung von Gelenk- oder Muskelbeschwerden im Bereich von Gelenken des menschlichen oder tierischen Körpers nach dem Oberbegriff des Anspruchs 1.

Es ist bekannt, Gelenkbeschwerden oder Muskelbeschwerden, die im Bereich eines Ellenbogens oder eines Kniegelenks auftreten, die häufig durch Überbeanspruchung oder Dauerbelastung entstehen, durch Verwendung einer das Gelenk übergreifenden Manschette zu mildern oder aufzuheben.

Wesentlich ist bei dieser Behandlungsmethode, dass eine Wirkung nur dann eintritt, wenn die Arm- oder Beinstreckung im Grenzbereich der Streckung entweder ganz unterbunden wird oder zumindest erschwert wird.

Bekannte, zu diesem Zweck verwendete Manschetten bestehen in der Regel aus zwei Manschettenteilen, von denen bei Verwendung an einem Arm ein Teil den Unterarm und ein zweiter Teil den Oberarm umgreift, wobei die beiden Teile in einem Übergangsbereich miteinander verbunden sind. Vor allem der den Unterarm umgreifende Teilbereich ist konisch ausgebildet, um der Armoberfläche möglichst eng angepasst folgen zu können. Die Größe der zu verwendenden Manschette hängt von der Körpergröße, insbesondere dem Armdurchmesser ab. Zur genauen Anpassung an den Körper ist die Manschette mit Befestigungsmitteln versehen, die häufig als Schnallen mit Dorn ausgebildet sind. Dadurch kann der Durchmesser der Manschettenteile dem Armdurchmesser angepasst werden.

In der EP 0 255 881 A1 ist eine entsprechende Bandage für die Behandlung von Unterarmbeschwerden offenbart. Bei dieser Bandage, die aus hautfreundlichem Leder ausgebildet ist, wird ein Kragen um den Unterarm gelegt und durch eine Schnalle im Durchmesser festgelegt. An dem oberen Rand des Kragens ist ein zweiter Kragen in Form einer Schlaufe befestigt, die um den Oberarm gelegt wird und einen Zuggurt zwischen unterem und oberem Kragen bildet. Bei Armstreckung verhindert der Zuggurt zwischen unterem und oberem Kragen eine vollständige Streckung des Arms und entlastet damit das Gelenk im gestreckten Zustand.

Da der Verbindungsbereich zwischen oberem und unterem Kragen den Zuggurt bildet und der obere Kragen als Schlaufe ausgebildet ist, ist es manchmal schwierig, den oberen Kragen ausreichend fest am Oberarm anzulegen, um ein Verrutschen zu verhindern. Bei einer zu festen Anlage wird der Tragekomfort jedoch eingeschränkt. Ferner ändert sich die Tragestellung des Oberarmkragens mit der Winkelstellung zwischen Oberarm und Unterarm, so dass es leicht zu Einschnürungen an einer Kante des Oberarmkragens kommen kann.

Aus der US 7,074,202 B1 ist eine Manschette zur orthetischen Behandlung von Gelenk- oder Muskelbeschwerden im Bereich eines Ellenbogens bekannt, welche ein um ein erstes Körperteil unterhalb des Gelenks gelegtes erstes Manschettenteil und ein zweites um ein zweites Körperteil oberhalb des Gelenks gelegtes Manschettenteil aufweist, wobei die Manschettenteile über einen Verbindungsbereich miteinander verbunden sind, welcher eine übermäßige Streckung der beiden Körperteile gegeneinander behindert, indem der Verbindungsbereich bei der Streckung unter Zugspannung gesetzt wird.

Die US 4,489,716 betrifft eine Orthese mit einer starren Aufnahmeschale zur Fixierung eines Ellenbogengelenks, bei der Haltegurte am Oberarm eine gewisse Flexibilität zur Verbesserung des Tragekomforts aufweisen.

Der Erfindung liegt daher die Aufgabe zu Grunde, den Tragekomfort einer Manschette zur orthetischen Behandlung von Gelenk- oder Muskelbeschwerden im Bereich von Gelenken des menschlichen oder tierischen Körpers derart zu verbessern, dass Fehlstellungen der Manschette in Bezug auf die Anlage am Körper vermieden werden und die Handhabung verbessert werden kann.

Diese Aufgabe wird durch die im Anspruch 1 angegebene Erfindung gelöst. Weitergehende Ausführungsformen der Erfindung sind in Unteransprüchen angegeben.

Die Erfindung geht aus von einer Manschette, wie sie in der EP 0 255 881 A1 angegeben ist.

Erfindungsgemäß ist wenigstens ein Teilbereich des oberhalb des Gelenks anzuordnenden zweiten Manschettenteils dehnbar ausgebildet, wobei der dehnbare Teilbereich aus zwei oder mehr voneinander getrennten bandförmigen Strängen des zweiten Manschettenteils gebildet ist, von denen der oder die der Gelenkbeuge abgewandten Stränge gummielastisch ausgebildet sind.

Die Dehnbarkeit des zweiten Manschettenteils führt dazu, dass die Winkelstellung des zweiten Manschettenteils an die jeweilige Winkelstellung zwischen den Körperteilen unterhalb und oberhalb des Gelenks angepasst ist. Dadurch verbessert sich der Tragekomfort erheblich und Einschnürungen des Oberarms an Kanten des Manschettenteils werden verhindert, so dass auch der Blutfluss im Oberarm nicht beeinträchtigt wird.

Vorzugsweise erstreckt sich der dehnbare Teilbereich quer über das zweite Manschettenteil, so dass der gesamte hintere Bereich des zweiten Manschettenteils flexibel ausgebildet ist.

In einer weiter gebildeten Ausführungsform kann von den bandförmigen Strängen des zweiten Manschettenteils jeweils ein Strang gummielastisch ausgebildet sein, während ein anderer Strang nicht längselastisch ist, um dadurch die Spannkraft des zweiten Manschettenteils erhalten zu können.

Das Innere der Manschettenteile enthält vorzugsweise eine Lage aus textilem, hautfreundlichem Material, insbesondere Velourstoff. Die Außenseite der Manschette kann über nahezu die gesamte Fläche mit zugfestem Flauschband versehen sein, das bei Verwendung von Klettbandverschlüssen den Gegenpart des Klettbandes bildet.

Die Manschette enthält möglichst wenig Schnallen oder andere Befestigungsteile, die von der Oberfläche der Manschette vorstehen können. Zur Umlenkung der Klettbefestigungszungen werden daher vorzugsweise flache D-förmige Ringe oder ringförmige Ösen verwendet. Alternativ dazu können auch Spalte in den Manschettenteilen verwendet werden, durch die die Befestigungszungen hindurchgeführt werden können.

Die Innenseiten der Manschettenteile können auch die Bequemlichkeit erhöhende Polsterungen aufweisen und ferner Pelotten, die auf bestimmte Körperteilbereiche eine erhöhte Druckkraft aufbringen können.

Um dem Nutzer eine klare Anweisung zu geben, wie er die Manschette anzulegen hat, weist diese vorzugsweise eine Markierung auf, die angibt, dass die Manschette so anzulegen ist, dass sich die Markierung im Bereich der Ellenbogenbeuge befindet.

Die Erfindung wird nachstehend unter Bezugnahme auf die Zeichnungen an einem Ausführungsbeispiel näher erläutert.

Es zeigen:
- Fig. 1: eine Vorderansicht einer Manschette,
- Fig. 2: eine erste Seitenansicht einer Manschette,
- Fig. 3: eine zweite Seitenansicht einer Manschette,
- Fig. 4: eine Rückansicht einer Manschette,
- Fig. 5: eine schräge Unteransicht einer Manschette,
- Fig. 6: eine Vorderansicht einer flachgelegten Manschette, und
- Fig. 7: eine Rückansicht einer flachgelegten Manschette.

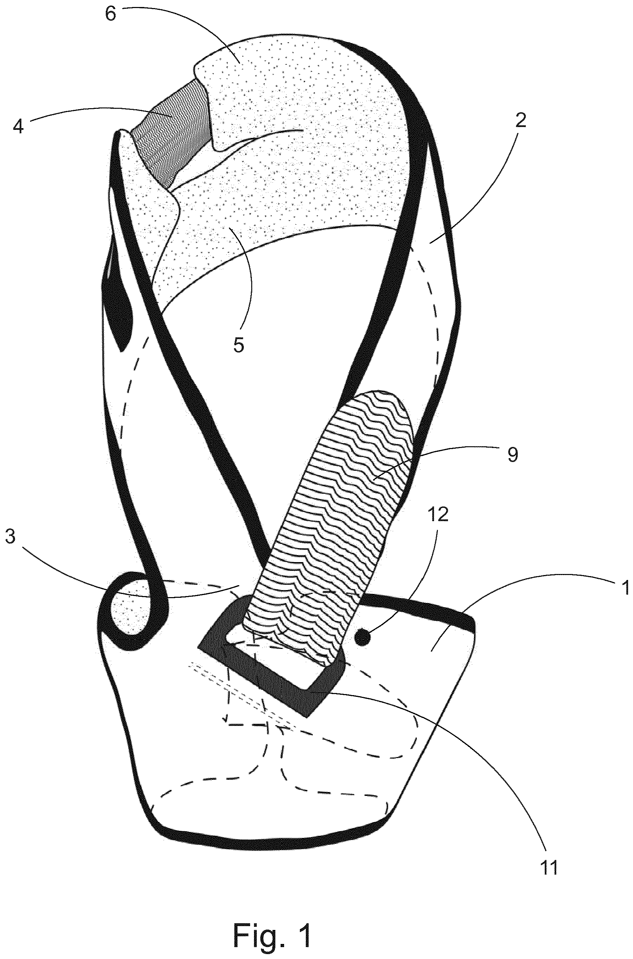

Die Manschette gemäß Fig. 1 dient in dem Ausführungsbeispiel der Stütze eines Ellenbogengelenks. Ein erstes Manschettenteil 1 mit einer konischen Gestalt wird dabei auf den Unterarm geschoben und zwar etwas unterhalb des Ellenbogengelenkes. Ein schlaufenförmiges zweites Manschettenteil 2 verläuft um den Oberarm. Die beiden Manschettenteile sind im Verbindungsbereich 3 miteinander verbunden. Dieser Bereich befindet sich in der praktischen Anwendung im Bereich der Ellenbogenbeuge. Um eine genaue Position der Manschette festzulegen, soll sich eine Markierung 12, die als farbige punktförmige Markierung auf dem ersten Manschettenteil sichtbar ist, im Bereich der Ellbogenbeuge befinden.

Die beiden Manschettenteile sind miteinander einstückig ausgebildet. Die das zweite Manschettenteil 2 bildende Schlaufe ist an einem Ende fest an dem ersten Manschettenteil 1 angeordnet. Das zweite Ende des zweiten Manschettenteils 2 weist einen Haft- oder Klettverschluss 9 auf, dessen zungenförmiges Ende durch einen D-förmigen Ring 11 geführt ist, der am ersten Manschettenteil 1 befestigt ist. Das Ende des zweiten Manschettenteils 2 kann damit durch den Klettverschluss in unterschiedlicher Stellung fixiert werden.

Das zweite Manschettenteil 2, das um den Oberarm gelegt wird, ist im oberen Bereich in zwei längslaufende Stränge 5 und 6 aufgeteilt. Während der Strang 5 längsstabil ausgebildet ist, befindet sich im Strang 6 ein Teilbereich 4, der längselastisch ausgeführt ist. Der Teilbereich 4 kann als gummielastisches Band ausgebildet sein. Die unterschiedliche Längselastizität der Stränge 5 und 6 führt zu dem Vorteil, dass unterschiedliche Winkelstellungen des zweiten Manschettenteils 2 gegenüber dem Oberarm problemlos ausgeglichen werden können, da die Druckkraft, die von dem zweiten Manschettenteil 2 auf den Oberarm ausgeübt wird, weitgehend unabhängig von der Winkelstellung in Bezug auf den Oberarm ist. Dadurch wird vermieden, dass sich der Teilbereich 4 in einer spitzwinkligen Stellung gegenüber der Oberarmlängsachse zu stark in den Oberarm eindrückt und insoweit Unwohlsein hervorrufen kann oder Druckstellen bildet.

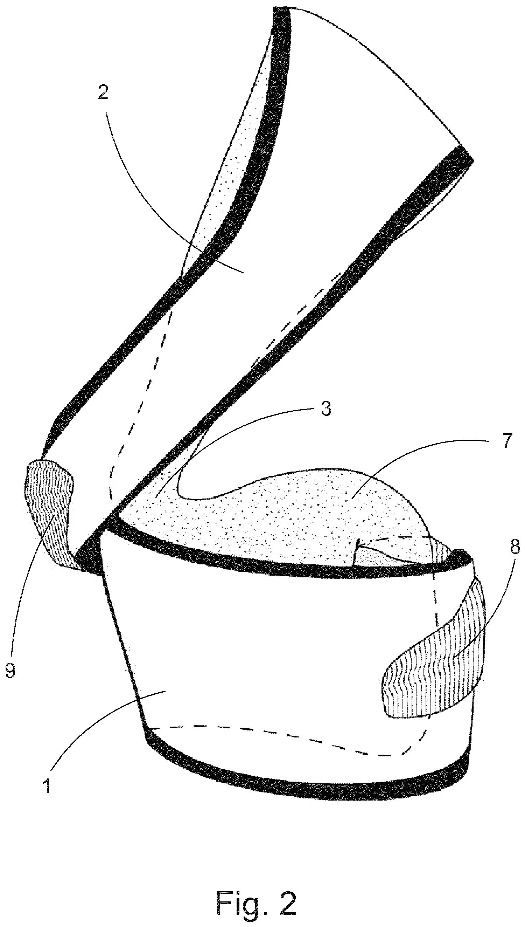

Fig. 2 zeigt die Manschette in Seitenansicht. Diese Figur dient insbesondere der Darstellung der Anordnung der Klettverschlüsse 8 und 9. Der Klettverschluss 9 ist entsprechend Fig. 1 ausgebildet. Ebenso ist das erste Manschettenteil mit einem Klettverschluss 8 versehen, über den der Durchmesser des ersten Manschettenteils eingestellt werden kann. In dieser Darstellung ist das zweite Manschettenteil 2 ohne Darstellung der Stränge 5 und 6 gezeigt.

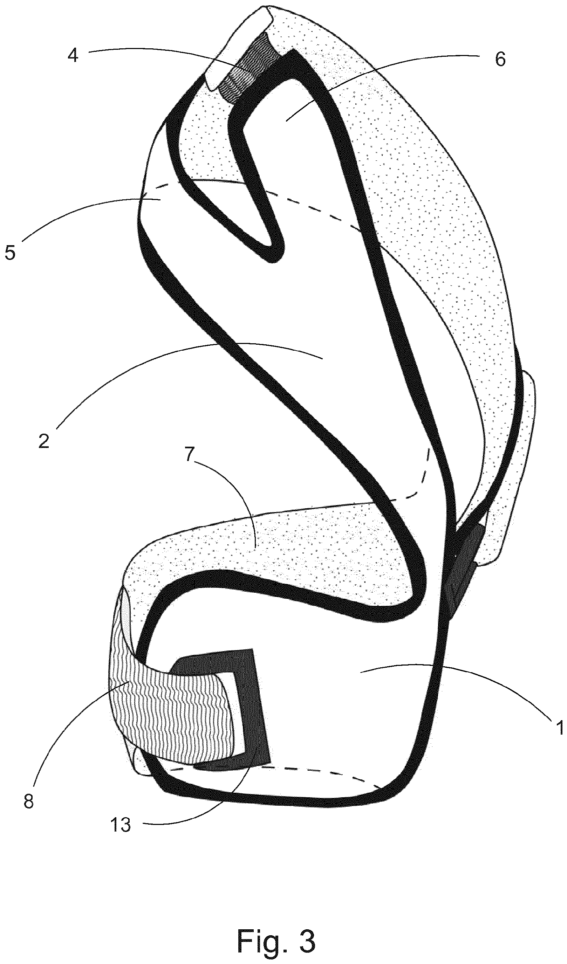

Fig. 3 zeigt eine zweite Seitenansicht der Manschette, in der die Anordnung der Stränge 5 und 6 mit dem im Strang 6 ausgebildeten längselastischen Teilbereich 4 dargestellt ist.

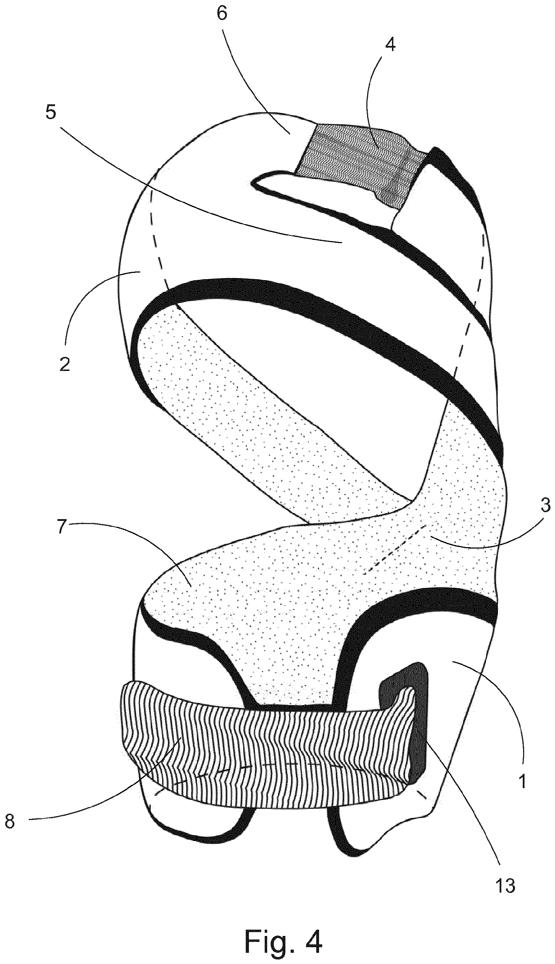

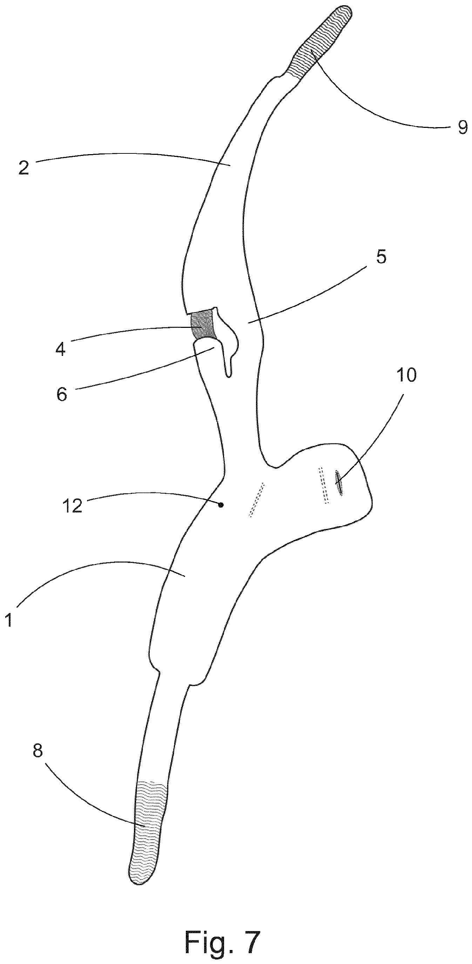

Fig. 4 zeigt eine Rückansicht mit dem Klettverschluss 8, dessen Zunge durch eine Schlitzöffnung 10 (Fig. 7) am anderen Ende des ersten Manschettenteils geführt ist. Sofern die Öffnung 10 eine ausreichende Randverstärkung aufweist, ist es nicht erforderlich, zusätzliche Befestigungselemente zu verwenden. Die Öffnung 10 kann allerdings auch durch einen weiteren D-förmigen Ring 13 oder eine ringförmige Öse verstärkt werden.

Die Innenseite der Manschette ist vorzugsweise mit einem textilartigen Material, insbesondere Velourstoff 7 belegt, der angenehme Trageeigenschaften aufweist. Die Außenseite der Manschette kann mit Flauschband belegt sein, welches das Gegenstück in dem Klettverschluss darstellt, so dass die Klettverschlusszungen an jeder Stelle der Außenseite der Manschette befestigt sein können.

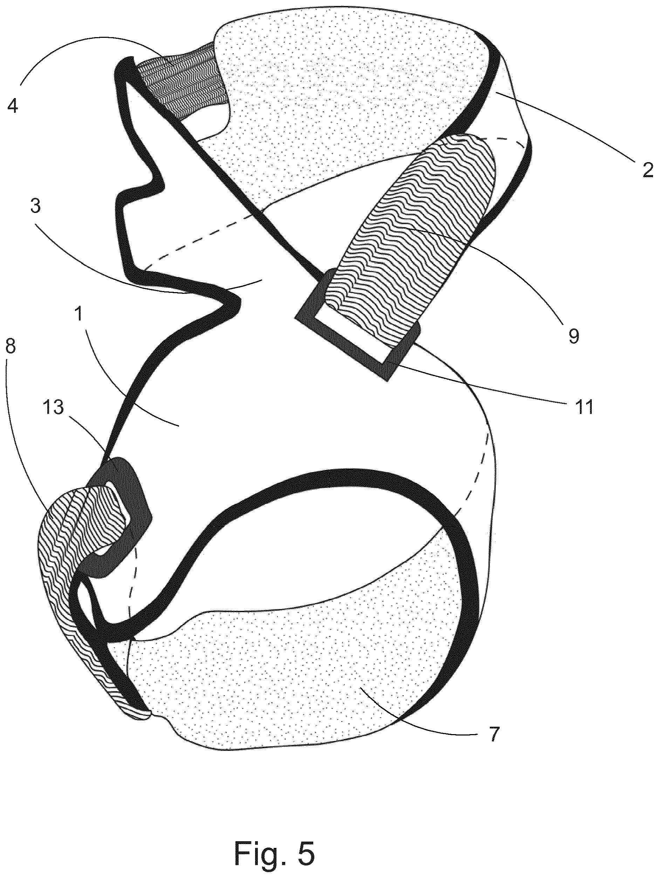

Fig. 5 zeigt eine Unteransicht zur weiteren Visualisierung der Manschette.

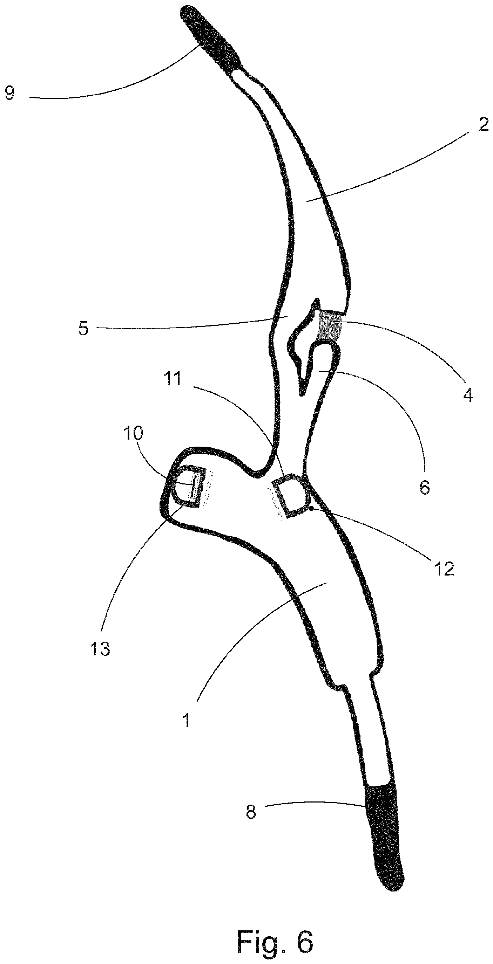

In Fig. 6 ist eine flachgelegte Manschette darstellt. An einem Ende des ersten Manschettenteils 1 befindet sich der Klettverschluss 8 und an seinem anderen Ende die mit einem D-förmigen Ring 13 verstärkte Öffnung 10. Die Figur zeigt deutlich, dass das zweite Manschettenteil 2 mit dem ersten Manschettenteil 1 fest verbunden ist. Die beiden Manschettenteile können einstückig ausgebildet sein, aus fertigungstechnischen Gründen kann jedoch auch vorgesehen sein, die beiden Manschettenteile über eine Nahtverbindung miteinander zu koppeln. Die Figur zeigt auch deutlich die Anordnung des flexiblen Teilbereichs 4 in dem Strang 6, während der Strang 5 nicht längselastisch ausgebildet ist. Der Haft- oder Klettverschluss 9 wird im benutzten Zustand durch den D-förmigen Ring 11 geführt.

Fig. 7 zeigt eine entsprechende Ansicht von Fig. 6 in rückwärtiger Ansicht.

Die Ausbildung der Manschette aus längsstabilem Material (ausgenommen der Teilbereich 4) erlaubt eine einfache und sichere Anlage an einen Arm, insbesondere einem Ellenbogen. Wenn die Manschette flach ausgebreitet ist, kann sie platzsparend gelagert oder verschickt werden. Das Anlegen an den Arm kann einhändig erfolgen, da kein kompliziertes Einfädeln durch Schnallen mit Dornen oder ähnlichem erforderlich ist. Die verwendeten Klettverschlüsse sind flexibel einstellbar und auch im angelegten Zustand justierbar.

### Bezugszeichen

- 1: erstes Manschettenteil
- 2: zweites Manschettenteil
- 3: Verbindungsbereich
- 4: Teilbereich
- 5: Strang
- 6: Strang
- 7: Velourstoff
- 8: Haft- oder Klettverschluss
- 9: Haft- oder Klettverschluss
- 10: Öffnung
- 11: Ring
- 12: Markierung
- 13: Ring

## Patentansprüche

1. Manschette zur orthetischen Behandlung von Gelenk- oder Muskelbeschwerden im Bereich von Gelenken des menschlichen oder tierischen Körpers, die gelenkübergreifend ein erstes Manschettenteil (1) zur Anlage an ein erstes Körperteil unterhalb des Gelenks und ein zweites Manschettenteil (2) zur Anlage an ein zweites Körperteil oberhalb des Gelenks aufweist, wobei die beiden Manschettenteile über einen Verbindungsbereich (3) miteinander verbunden sind und jeweils aus einem mit Befestigungsmitteln (8, 9) versehenen um das jeweilige Körperteil legbaren Gurt gebildet sind, wobei der Verbindungsbereich (3) zwischen den beiden Manschettenteilen bei Anlage der Manschette an den Körper im Bereich der Innenseite des Gelenks eine Streckung des ersten Körperteils gegenüber dem zweiten Körperteil behindert, indem der Verbindungsbereich (3) unter Zugspannung versetzt wird, **dadurch gekennzeichnet, dass** wenigstens ein Teilbereich (4) des zweiten Manschettenteils (2) oberhalb des Gelenks dehnbar ausgebildet ist, wobei der dehnbare Teilbereich (4) aus zwei oder mehr voneinander getrennten bandförmigen Strängen (5, 6) des zweiten Manschettenteils (2) gebildet ist, von denen der oder die der Gelenkbeuge abgewandten Stränge (6) gummielastisch ausgebildet sind.

2. Manschette nach Anspruch 1, **dadurch gekennzeichnet, dass** der dehnbare Teilbereich (4) sich quer über das zweite Manschettenteil (2) erstreckt.

3. Manschette nach Anspruch 1 oder 2, **dadurch gekennzeichnet, dass** die Manschettenteile innenseitig eine Lage aus textilem Material, insbesondere Velourstoff (7) enthalten.

4. Manschette nach einem oder mehreren der vorhergehenden Ansprüche, **dadurch gekennzeichnet, dass** die Befestigungsmittel als einstellbare Haft- oder Klettverschlüsse (8, 9) ausgebildet sind, wobei jeweils eine Zunge eines ersten Endes des jeweiligen Manschettenteils durch eine ringförmige Öse oder einen D-förmigen Ring (11, 13) oder eine Öffnung (10) am zweiten Ende des jeweiligen Manschettenteils geführt werden kann und mit sich selbst oder einem Oberflächenteil des jeweiligen Manschettenteils verbindbar ist.

5. Manschette nach einem oder mehreren der vorhergehenden Ansprüche, **dadurch gekennzeichnet, dass** die äußere Oberfläche der Manschettenteile (1, 2) als zugfestes Flauschband ausgebildet ist, das die Außenseite der Manschette wenigstens teilweise bedeckt.

6. Manschette nach Anspruch 4, **dadurch gekennzeichnet, dass** sich der Ring (11) oder die Öffnung (10) für die Befestigung des zweiten Manschettenteils (2) an dem ersten Manschettenteil (1) im Verbindungsbereich (3) zwischen erstem und zweitem Manschettenteil befindet.

7. Manschette nach einem oder mehreren der vorhergehenden Ansprüche, **dadurch gekennzeichnet, dass** die am Körper anliegende Innenseite der Manschettenteile mit einem oder mehreren gepolsterten Abschnitten versehen ist.

8. Manschette nach einem oder mehreren der vorhergehenden Ansprüche, **dadurch gekennzeichnet, dass** das erste Manschettenteil (1) eine außenseitige Markierung (12) zur Bestimmung des Ortes des im Bereich der Gelenkbeuge anzuordnenden Verbindungsbereichs aufweist.

## Claims

1. Cuff for the orthotic treatment of joint or muscle problems in the region of joints of the human or animal body, which cuff comprises by overlapping the joint a first cuff part (1) for abutment against a first body part below the joint and a second cuff part (2) for abutment against a second body part above the joint, the two cuff parts being connected to one another via a connecting area (3) and each being formed from a belt which can be placed around the respective body part and is provided with fastening means (8, 9), whereas, when applying the cuff to the body in the area of the inner side of the joint, the connecting area (3) between the two cuff parts hinders stretching of the first body part relative to the second body part, by placing the connecting area (3) under tensile stress, **characterized in that** at least one section (4) of the second cuff part (2) above the joint is designed as stretchable, the stretchable section (4) being formed from two or more strip-shaped strands (5, 6) of the second cuff part (2) which are separated from one another and of which the strand or strands (6) facing away from the bend of the joint is or are of rubber-elastic design.

2. Cuff according to claim 1, **characterized in that** the extensible section (4) extends transversely over the second cuff part (2).

3. Cuff according to claim 1 or 2, **characterised in that** the cuff parts contain a layer of textile material, in particular velour fabric (7), on their inner surface.

4. Cuff according to one or more of the preceding claims, **characterised in that** the fastening means are constructed as adjustable adhesive or Velcro fasteners (8, 9), wherein in each case a tongue of a first end of the respective cuff part can be guided through an annular eyelet or a D-shaped ring (11, 13) or an opening (10) at the second end of the respective cuff part and can be connected to itself or a surface part of the respective cuff part.

5. Cuff according to one or more of the preceding claims, **characterized in that** the outer surface of the cuff parts (1, 2) is formed as a tension-resistant fleece band which at least partially covers the outer side of the cuff.

6. Cuff according to claim 4, **characterized in that** the ring (11) or the opening (10) for fastening the second cuff part (2) to the first cuff part (1) is located in the connecting area (3) between the first and second cuff parts.

7. Cuff according to one or more of the preceding claims, **characterized in that** the inside of the cuff parts adjacent to the body is provided with one or more padded portions.

8. Cuff according to one or more of the preceding claims, **characterised in that** the first cuff part (1) has an external marking (12) for determining the location of the connecting area to be arranged in the region of the bend of the joint.

## Revendications

1. Manchette pour le traitement orthétique de troubles articulaires ou musculaires dans la zone d'articulations du corps humain ou animal, qui comporte, couvrant une articulation, une première partie de manchette (1) à appliquer sur une partie du corps au-dessous de l'articulation, et une deuxième partie de manchette (2) à appliquer sur une deuxième partie du corps au-dessus de l'articulation, dans laquelle les deux parties de manchette sont reliées entre elles par une zone de liaison (3) et sont formées chacune d'une sangle pourvue de moyens de fixation (8, 9) et apte à être posée autour de la partie du corps correspondante, dans laquelle la zone de liaison (3) entre les deux parties de manchette, lorsque la manchette est appliquée sur le corps, gêne dans la zone du côté intérieur de l'articulation un allongement de la première partie du corps par rapport à la deuxième partie du corps, du fait que la zone de liaison (3) est soumise à une contrainte de traction, **caractérisée en ce qu'**au moins une zone partielle (4) de la deuxième partie de manchette (2) au-dessus de l'articulation est extensible, dans laquelle la zone partielle extensible (4) est formée de deux tronçons ou plus (5, 6) en forme de bandes et séparés de la deuxième partie de manchette (2), parmi lesquels le ou les tronçons (6) opposés à la pliure de l'articulation sont élastiques comme du caoutchouc.

2. Manchette selon la revendication 1, **caractérisée en ce que** la zone partielle extensible (4) s'étend transversalement sur la deuxième partie de manchette (2).

3. Manchette selon la revendication 1 ou 2, **caractérisée en ce que** les parties de manchette contiennent, côté intérieur, une couche de matériau textile, en particulier du tissu velouté (7).

4. Manchette selon l'une au moins des revendications précédentes, **caractérisée en ce que** les moyens de fixation sont formés comme des fermetures par adhérence ou par crochets et boucles réglables (8, 9), dans laquelle une languette d'une première extrémité de la partie de manchette respective peut passer à travers un œillet annulaire ou un anneau en forme de D (11, 13) ou une ouverture (10) sur la deuxième extrémité de la deuxième partie de manchette respective et peut être reliée à elle-même ou à un élément de surface de la partie de manchette respective.

5. Manchette selon l'une au moins des revendications précédentes, **caractérisée en ce que** la surface extérieure des parties de manchette (1, 2) est formée comme une bande velours résistante à la traction qui couvre au moins partiellement le côté extérieur de la manchette.

6. Manchette selon la revendication 4, **caractérisée en ce que** l'anneau (11) ou l'ouverture (10) pour la fixation de la deuxième partie de manchette (2) à la première partie de manchette (1) se trouve dans la zone de liaison (3) entre les première et deuxième parties de manchette.

7. Manchette selon l'une au moins des revendications précédentes, **caractérisée en ce que** le côté intérieur des parties de manchette appliqué sur le corps est pourvu d'une ou plusieurs sections rembourrées.

8. Manchette selon l'une au moins des revendications précédentes, **caractérisée en ce que** la première partie de manchette (1) présente un marquage côté extérieur (12) pour déterminer l'endroit de la zone de liaison à disposer dans la zone de la pliure de l'articulation.
